Europäisches Patentamt

European Patent Office

Office européen des brevets

(19)

(11) Veröffentlichungsnummer: **0 133 938**
**B1**

(12) # EUROPÄISCHE PATENTSCHRIFT

(45) Veröffentlichungstag der Patentschrift:
05.11.86

(51) Int. Cl.⁴: **C 07 C 85/18,** C 07 C 87/06

(21) Anmeldenummer: **84108454.4**

(22) Anmeldetag: **18.07.84**

(54) **Verfahren zur Herstellung von Aminen.**

(30) Priorität: 27.07.83 DE 3327000

(43) Veröffentlichungstag der Anmeldung:
13.03.85 Patentblatt 85/11

(45) Bekanntmachung des Hinweises auf die Patenterteilung:
05.11.86 Patentblatt 86/45

(84) Benannte Vertragsstaaten:
AT BE CH DE FR GB IT LI NL

(56) Entgegenhaltungen:
EP-A-0 077 016
DE-A-2 830 787
US-A-4 307 250
US-A-4 375 002

**Die Akte enthält technische Angaben, die nach
dem Eingang der Anmeldung eingereicht wurden
und die nicht in dieser Patentschrift enthalten sind.**

(73) Patentinhaber: BASF Aktiengesellschaft, Carl-
Bosch- Strasse 38, D-6700 Ludwigshafen (DE)

(72) Erfinder: Taglieber, Volker, Dr., Franz- Liszt-
Strasse 15, D-6904 Eppelheim (DE)
Erfinder: Hoelderich, Wolfgang, Dr., Mannheimer
Strasse 18C, D-6710 Frankenthal (DE)
Erfinder: Kummer, Rudolf, Dr., Kreuzstrasse 6,
D-6710 Frankenthal (DE)
Erfinder: Mross, Wolf Dieter, Dr., Anselm-
Feuerbach- Strasse 21, D-6710 Frankenthal (DE)
Erfinder: Saladin, Guenter, Muenchbuschweg 73,
D-6700 Ludwigshafen (DE)

**Beschreibung**

Die Erfindung betrifft ein Verfahren zur Herstellung von Aminen durch Anlagerung von Ammoniak oder analog reagierenden Aminen an Olefine.

Bei der Addition von Ammoniak oder analog reagierenden Aminen an Olefine handelt es sich um eine seit langem bekannte und vielfach beschriebene Reaktion. Eine technische Realisierung ist bislang nicht erfolgt, da nach den bekannten Verfahren nur ungenügende Selektivitäten und/oder Katalysator-Standzeiten erreicht werden, wie beispielsweise nach US-PS 2 623 061, US-PS 2 422 631, US-PS 2 501 556 und US-PS 3 412 158. Auch das in US-PS 4 307 250 beschriebene Verfahren eignet sich nicht für die Durchführung im technischen Maßstab. Die als Katalysator verwendeten Alumino-Zeolithe begünstigen starke Polymerbildung und nachfolgende Verkokung, die den Katalysator schnell desaktiviert.

Es wurde nun gefunden, daß man hohe Standzeiten und hohe Selektivität der Katalysatoren bei der Herstellung von Aminen aus Olefinen und Ammoniak oder primären oder sekundären Aminen bei Temperaturen zwischen 80°C und 400°C und bei Drücken zwischen 40 und 700 bar in Gegenwart eines zeolithischen Katalystors erzielt, wenn man einfach und/oder mehrfach ungesättigte Olefine mit 2-10 C-Atomen bzw. deren Mischungen und Ammoniak oder primäre oder sekundäre Amine oder Gemische derselben in Gegenwart eines Borosilikatoder Borogermanatzeolithen des Pentasil-Typs als Katalysator umsetzt, das erhaltene Amin abtrennt und die nichtumgesetzten Einsatzstoffe zurückführt.

Das erfindungsgemäße Verfahren zeichnet sich dadurch aus, daß bereits mit niedrigem Ammoniak- bzw. Amin-Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine zusammen mit Olefinen im molaren Verhältnis von 1:1 bis 5:1 gemischt einem Festbett- oder Wirbelbettreaktor zuführt und bei einem Druck von 40 bis 700 bar, insbesondere 200 bis 300 bar und einer Temperatur von 80 bis 400°C, insbesondere von 250 bis 350°C in der Gasphase oder im überkritischen Zustand unsetzt. Eine andere Ausführungsform besteht darin, daß die Reaktion in Flüssigphase bei einem Druck von 40 bis 80 bar und einer Temperatur von 60°C bis 120°C in einem Rührkessel, einem Fest-/ Flüssigkeit-Fließbett oder einem Strömungsrohr durchgeführt wird.

Aus dem Reaktionsaustrag wird das gewünschte Produkt mit Hilfe bekannter Methoden, beispielsweise Destillation oder Extraktion, erhalten und nötigenfalls mittels weiterer Trennoperationen auf die gewünschte Reinhelt gebracht. Die nichtumgesetzten Eingangsstoffe werden in den Reaktor zurückgeführt.

Man kann einfach oder mehrfach ungesättigte Olefine mit 2 bis 10 C-Atomen bzw. deren Mischungen als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstigt das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Die Lage des Temperaturoptimums bezüglich Umsatz und Selektivität ist von der Konstitution des Olefins, des eingesetzten Amins und des Katalysators abhängig und liegt meist im Bereich von 250°C bis 350°C. Die Verweilzeit ist abhängig von den Einsatzstoffen und liegt zweckmäßigerweise im Bereich zwischen Bruchteilen einer Sekunde und wenigen Minuten.

Als Katalysatoren für die Aminierung von Olefinen verwendet man Borosilikat- oder Borogermanat-Zeolithe des Pentasiltyps.

Geeignete Borosilikatzeolithe bzw. Borogernanatzeolithe werden bei 90 bis 170°C unter autogenem Druck synthetisiert, indem man eine Borverbindung, z.B. $H_3BO_3$ mit einer Siliciumverbindung oder ·einer Germaniumverbindung, vorzugsweise hochdisperses Siliciumdioxid und Germaniumoxid in wäßriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt.

Derartige Borosilikatzeolithe oder Borogermanatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. Methanol bzw. 1,4-Butandiol durchführt.

Die so hergestellten Borosilikatzeolithe und Borogermanatzeolithe kann man nach ihrer Isolierung, Trocknung bei 100 bis 160°C, vorzugsweise bei etwa 110°C und Calcination bei 450 bis 550°C, vorzugsweise bei etwa 500°C, mit einem Bindemittel im Verhältnis 90:10 bis 40:60 Gew.-% zu Strängen oder Tabletten verformen. Als Bindemittel eignen sich verschiedene Aluminiumoxide, bevorzugt Boehmit, amorphe Aluminosilikate mit einem $SiO_2/Al_2O_3$-Verhältnis von 25:75 bis 95:5, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Presslinge zweckmäßig bei 110°C/16 h getrocknet und bei 500°C/16 h calciniert.

Eine besondere Ausführungsform besteht darin, daß der isolierte Borosilikat- und/oder Borogermantzeolith direkt nach der Trocknung verformt wird und erstmals nach der Verformung einer Calcination unterworfen wird.

2

Aus dem zu Strängen verformten Katalysator kann man durch Mahlen und Sieben Wirbelgut in der Größe von 0,1 bis 0,8 mm erhalten.

Nach Desaktivierung der zeolithischen Katalysatoren durch Koksabscheidung während der erfindungsgemäßen Umsetzung kann man die Katalysatoren durch Abbrennen der Koksablagerung mit Luft oder mit einem Luft/N$_2$-Gemisch bei 400°C bis 550°C, bevorzugt bei etwa 500°C, in einfacher Weise regenerieren. Sie erhalten dadurch ihre Anfangsaktivität zurück.

Zur Erhöhung der Selektivität, der Standzeit und der Anzahl der möglichen Regenerierungen kann man verschiedene Modifizierungen an zeolithischen Katalysatoren vornehmen.

Eine Modifizierung der Katalysatoren besteht darin, daß man die unverformten oder die verformten Zeolithe mit Alkalimetallen wie Na und K, Erdalkalimetallen wie Ca, Mg, Erdmetallen wie Tl, Übergangsmetallen wie Mn, Fe, Mo, Cu, Zn und seltene Erdmetalle wie La, Ce ionenaustauschen bzw. dotieren kann.

Eine vorteilhafte Ausführungsform besteht darin, daß man die verformten Pentasilzeolithe in einem Strömungsrohr vorlegt und bei 20 bis 100°C, z.B. ein Halogenid oder ein Nitrat der oben beschriebenen Metalle darüberleitet. Ein derartiger Ionenaustausch kann z.B. an der Wasserstoff-, Ammonium- und Alkaliform der Zeolithe vorgenommen werden.

Eine andere Möglichkeit der Metallaufbringung auf die Zeolithe besteht darin, daß man das zeolithische Material, z.B. mit einem Halogenid, einem Nitrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Zeolithen kann eine Nachbehandlung mit Wasserstoff und/ oder mit Wasserdampf günstig sein.

Eine weitere Möglichkeit der Modifizierung besteht darin, daß man das zeolithische Material - verformt oder unverformt - einer Behandlung mit Säurenü wie Salzsäure-, Flußsäure- und Phosphorsäure unterwirft.

Eine besondere Ausführungsform besteht darin, daß man das zeolithische Pulver vor seiner Verformung mit Flußsäure 0,001 n bis 2 n, bevorzugt 0,05 bis 0,5 n 1 bis 3 Stunden unter Rückfluß behandelt. Nach Abfiltrieren und Auswaschen wird bei 100 bis 160°C getrocknet und bei 400 bis 550°C calciniert. Eine weitere besondere Ausführungsform liegt in einer HCl-Behandlung der Zeolithe nach ihrer Verformung mit Bindemittel. Hierbei wird der Zeolith 1 bis 3 Stunden zwischen 60 und 80°C mit einer 3 bis 25%igen, insbesondere mit einer 12 bis 20%igen Salzsäure behandelt, anschließend ausgewaschen, bei 100 bis 160°C getrocknet und bei 400 bis 550°C calciniert.

Eine andere Möglichkeit der Modifizierung ist gegeben durch einen Austausch mit Ammonsalzen, z.B. mit NH Cl oder mit Mono-, Di- oder Polyaminen. Hierbei wird der in der H-Form oder einer anderen Ammoniumform vorliegende, mit Bindemittel verformte Zeolith bei 60 bis 80°C mit 10 bis 25%iger, bevorzugt 20%iger NH Cl-Lösung 2 h kontinuierlich in gewichtsmäßiger Zeolith/Ammonchlorid-Lösung von 1:15 ausgetauscht und danach bei 100 bis 120°C getrocknet.

Die Katalysatoren kann man als 2 mm bis 4 mm Stränge, als Tabletten mit 3 bis 5 mm Durchmesser oder als Wirbelgut mit einer Größe von 0,1 bis 0,8 mm oder als Split mit 0,5 - 1 mm Durchmesser für die Aminierung der Olefine einsetzen.

Die nachfolgenden Beispiele veranschaulichen die Erfindung.

## Beispiel 1

Katalysator A wird in einer hydrothermalen Synthese aus 64 g SiO$_2$ (hochdisperse Kieselsäure) 12,2 g H$_3$BO$_3$, 800 g einer wäßrigen 1,6-Hexandiamin-Lösung (Mischung 50:50 Gew.-%) bei 170°C unter autogenem Druck in einem Rührautoklaven hergestellt. Nach Abfiltrieren und Auswaschen wird das kristalline Reaktionsprodukt bei 110°C/24 h getrocknet und bei 500°C/24 h calciniert. Man erhält einen Borosilikatzeolithen vom Pentasiltyp, der 94,2 Gew.-% SiO$_2$ und 2,32 Gew.-% B$_2$O$_3$ enthält.

Aus diesem Zeolith werden durch Verformen mit Boehmit im Gewichtsverhältnis 60:40 2 mm-Stränge hergestellt, die bei 110°C/16 h getrocknet und bei 500°C/24 h calciniert werden.

Katalysator B wird durch Behandlung von 50 g des oben beschriebenen Borosilikatzeolithen mit 140 ml 0,1n HF unter Rückfluß während 1 h hergestellt. Nach der Filtration und Auswaschen mit Wasser wird bei 110°C/16 h getrocknet und bei 500°C/5 h calciniert. Dieses Produkt wird mit Boehmit im Verhältnis 60:40 verstrangt, bei 110°C/16 h getrocknet und bei 500°C/16 h calciniert.

Katalysator C wird erhalten, indem an Katalysator A ein Ionenaustausch mit einer Na-Verbindung bei RT vorgenommen wird, so daß der Katalysator nach Auswaschen mit Wasser und Trocknung bei 110°C/16 h einen Na-Gehalt von 0,32 Gew.-% aufweist.

In einen 0,3 l-Rührautoklaven werden jeweils 10 ml der oben beschriebenen Katalysatoren A, B oder C gegeben und nach dem Verschließen die Olefine und Ammoniak bzw. Amine aufgedrückt. Die Menge an Einsatzprodukt wird so bemessen, daß bei der gewählten Reaktionstemperatur der gewünschte Druck als Eigendruck der Reaktionspartner erreicht wird. Das molare Verhältnis von Ammoniak/Amin zu Olefin wird von 1:1 bis 5:1 variiert, die Reaktionsdauer auf 30 min. festgelegt.

Der Austrag wurde getrennt nach Flüssig- und Gasphase gaschromatographisch untersucht. Die in Tabelle 1 aufgeführten Umsätze beziehen sich immer auf das Olefin; die angegebenen Selektivitäten beziehen sich auf

3

die Hauptprodukte: Ethylamin aus Ethylen, Isopropylamin aus Propylen, Isobutylamin aus Buten-1, tertiär Butylamin aus Isobuten, Isopentylamin aus Isobuten mit Methylanin und 1-Amino-4(1-aminoethyl)cyclohexan aus 4-Vinyl-1-cyclohexen.

**Tabelle 1**

| Olefin | Amin | Amin:Olefin [mol/mol] | Katalysator | Temperatur [°C] | Druck [bar] | Umsatz [%] | Selektivität [%] |
|---|---|---|---|---|---|---|---|
| Ethylen | $NH_3$ | 2:1 | A | 350 | 295 | 4,7 | 97,8 |
| " | " | " | A | 370 | 305 | 7,3 | 94,0 |
| " | " | " | C | 370 | 290 | 6,9 | 95,2 |
| Propylen | " | 1,5:1 | A | 330 | 285 | 9,2 | 96,4 |
| " | " | " | A | 350 | 290 | 11,3 | 97,8 |
| " | " | " | C | 330 | 270 | 10,1 | 95,9 |
| " | " | 3:1 | C | 350 | 305 | 11,7 | 94,7 |
| Buten-1 | " | 1,5:1 | A | 330 | 300 | 10,9 | 98,2 |
| Isobuten | " | " | A | 300 | 280 | 12,3 | 97,2 |
| " | " | " | A | 330 | 570 | 13,7 | 98,5 |
| " | " | " | A | 350 | 295 | 14,1 | 95,7 |
| " | " | " | B | 330 | 300 | 13,9 | 96,8 |
| " | " | " | C | 330 | 275 | 13,1 | 97,4 |
| " | $CH_3NH_2$ | 2:1 | A | 330 | 235 | 9,3 | 93,2 |
| 4-Vinyl-1-cyclohex-an | $NH_3$ | 4:1 | A | 330 | 260 | 8,7 | 87,6 |

**Beispiel 2**

Für eine kontinuierliche Herstellung wird ein Hochdruck-Reaktor mit 2 m Länge und 24 mm Innendurchmesser eingesetzt, der mittels einer Alublockheizung beheizt und mit dreifacher Innentemperaturmessung sowie mit einer Druckhaltung ausgestattet ist. Es werden jeweils 60 ml Katalysator eingebaut und der obere Teil des Reaktorrohres mit Porzellanringen gefüllt. Der Zulauf von Olefin und Amin erfolgt von oben.

Die Analyse der Reaktorausträge wird gaschromatographisch und z.T. zusätzlich destillativ durchgeführt.

Die Ergebnisse mit den verschiedenen Katalysatoren sind in Tabelle 2 wiedergegeben. Einige Ergebnisse wurden ohne zwischenzeitliche Regenerierung des Katalysators erhalten. Die Analyse des nach 28 Tagen ununterbrochener Betriebsdauer ausgebauten Katalysators ergibt einen Kohlenstoffgehalt von 11,0 %.

**Tabelle 2**

| Olefin | Amin | Amin:Olefin [mol/mol] | Katalysator | Katalysator-Belastung | Tempe-ratur [°C] | Druck [bar] | Umsatz [%] | Selekt. [%] | RZA [kg/l.K.h] |
|---|---|---|---|---|---|---|---|---|---|
| Propylen | $NH_3$ | 1,5:1 | A | 3 | 350 | 300 | 7,8 | 98,7 | 0,23 |
| " | " | " | A | 9 | 350 | 300 | 5,3 | 98,3 | 0,47 |
| " | " | " | A | 18 | 350 | 300 | 3,9 | 99,0 | 0,70 |
| " | " | " | A | 9 | 370 | 300 | 6,4 | 96,4 | 0,55 |
| Isobuten | " | " | A | 9 | 300 | 300 | 5,4 | 99,7 | 0,38 |
| " | " | " | A | 9 | 310 | 300 | 8,6 | 99,8 | 0,65 |
| " | " | " | A | 9 | 320 | 300 | 6,3 | 99,6 | 0,33 |
| " | " | " | A | 18 | 350 | 300 | 4,7 | 98,0 | 0,69 |
| " | " | " | A | 9 | 320 | 200 | 4,9 | 99,4 | 0,26 |

RZA = Raum-Zeit-Ausbeute Isopropylamin bzw. tertiär Butylamin

## Beispiel 3

Katalysator D wird erhalten, inden man 50 g des Katalysators A mit 250 ml 15%iger Salzsäure unter Rückfluß 1 h behandelt, nach der Filtration Cl-frei wäscht, bei 110°C/16 h trocknet und bei 500°C/5h calciniert.

Katalysator E wird durch Imprägnierung von 50 g des Katalysators A mit 9,4 g $Zn(NO_3)_3.6H_2O$ und anschließender Calcination bei 540°C/2h hergestellt.

Katalysator F erhält man, indem der in Beispiel 1 beschriebene Borosilikatzeolith mit einem Gemisch aus hochdispersem $SiO_2$ und $Al_2O_3$ ($Al_2O_3$-Ge-halt = 3,5 Gew.%) im Gewichtsverhältnis 60:40 zu Strängen verformt, bei 110°C/16 h getrocknet und bei 500°C/16 h calciniert wird.

Katalysator G erhält man, indem der in Beispiel 1 beschriebene Borosilikatzeolith mit einem Gemisch aus 90 Gew.% hochdispersem $SiO_2$ und 10 Gew.% Boehmit im Gewichtsverhältnis 60:40 zu Strängen verformt, bei 110°C/16 h getrocknet und bei 500°C/16 h calciniert wird.

Im einem Rohrreaktor (6 mm Innendurchmesser) wird unter isothermen Bedingungen bei 300°C und einem Druck von 300 bar ein Gemisch aus Isobuten und Ammoniak im molaren Verhältnis von 1:1,3 an den Katalysatoren A, B, C, D, E, F und G diskontinuierlich umgesetzt. Die Reaktionsprodukte werden im Caschromatographen analysiert.

Die Versuchsergebnisse sind in Tabelle 3 zusammengefaßt.

### Tabelle 3

| Katalysator | A | A | B | C | D | E | F | G |
|---|---|---|---|---|---|---|---|---|
| Temperatur [°C] | 300 | 300 | 300 | 300 | 300 | 300 | 300 | 300 |
| Druck [bar] | 300 | 300 | 300 | 300 | 300 | 300 | 300 | 300 |
| GHSV $\left[\frac{1\ \text{Edukt}}{\text{g Kat.h}}\right]$ | 4 | 6,5 | 4,5 | 10 | 15 | 3,3 | 11 | 3,3 |
| Umsatz von Isobuten | 13,1 | 12,8 | 14,5 | 13,6 | 7,8 | 13,6 | 17,3 | 15,0 |
| Selektivität tert.-Butylamin | 96,9 | 96,6 | 95,7 | 96,9 | 92,2 | 95,5 | 97,3 | 97,8 |

## Patentansprüche

1. Verfahren zur Herstellung von Aminen aus Olefinen und Ammoniak oder primären oder sekundären Aminen bei Temperaturen zwischen 80°C und 400°C bei Drücken zwischen 40 und 700 bar in Gegenwart eines zeolithischen Katalysators, dadurch gekennzeichnet, daß man einfach und/oder mehrfach ungesättigte Olefine mit 2 bis 10 C-Atomen bzw. deren Mischungen und Ammoniak oder primäre oder sekundäre Amine oder Gemische derselben in Gegenwart eines Borosilikat- oder Borogermanat-Zeolithen des Pentasil-Typs als Katalysator umsetzt, das erhaltene Amin abtrennt und die nichtumgesetzten Einsatzstoffe zurückführt.

2. Verfahren nach Anspruch 1, dadurch gekennzeichnet, daß der Katalysator mit einem Bindemittel verformt und anschließend calciniert wird.

3. Verfahren nach Anspruch 1 und 2, dadurch gekennzeichnet, daß der Katalysator einer Säurebehandlung unterworfen wird.

4. Verfahren nach Anspruch 1 bis 3, dadurch gekennzeichnet, daß der Katalysator mit Übergangsmetallen dotiert wird.

5. Verfahren nach Anspruch 1 bis 3, dadurch gekennzeichnet, daß der Katalysator mit Seltenen Erden dotiert wird.

6. Verfahren nach Anspruch 1 bis 3, dadurch gekennzeichnet, daß der Katalysator mit Alkali-, Erdalkali- und/oder Erdmetallen dotiert wird.

7. Verfahren nach Anspruch 1 bis 3, dadurch gekennzeichnet, daß der Katalysator nach Behandlung mit Ammonsalzen in seiner Ammoniumforn eingesetzt wird.

## Claims

1. A process for the preparation of an amine from an olefin and ammonia or a primary or secondary amine at from 80 to 400°C and under from 40 to 700 bar in the presence of a zeolite catalyst, wherein a monounsaturated or polyunsaturated olefin of 2 to 10 carbon atoms or a mixture thereof is reacted with ammonia or a primary or secondary amine, or a mixture thereof, in the presence of a borosilicate or borogermanate zeolite of the pentasil type as catalyst, the resulting amine is isolated, and the unreacted starting materials are recycled.

2. A process as claimed in claim 1, wherein the catalyst is molded together with a binder and then calcined.

3. A process as claimed in claims 1 and 2, wherein the catalyst is treated with an acid.

4. A process as claimed in claims 1 to 3, wherein the catalyst is doped with a transition metal.

5. A process as claimed in claims 1 to 3, wherein the catalyst is doped with a rare earth.

6. A process as claimed in claims 1 to 3, wherein the catalyst is doped with an alkali metal, an alkaline earth metal and/or an earth metal.

7. A process as claimed in claims 1 to 3, wherein the catalyst is treated with an ammonium salt and then used in its ammonium form.

**Revendications**

1. Procédé de préparation d'amines à partir d'oléfines et d'amoniac ou d'amines primaires ou secondaires, à des températures comprises entre 80°C et 400°C, sous des pressions comprises entre 40 et 700 bars et en présence d'un catalyseur zéolitique, caractérisé en ce qu' on fait réagir des oléfines à 2 - 10 atomes de C, présentant une et/ou plusieurs insaturations ou leurs mélanges et l'ammoniac ou des amines primaires ou secondaires ou des mélanges de celles-ci, en présence d'une zéolite de borosilicate ou de borogermanate du type pentasile servant de catalyseur, on sépare l'amine obtenue et on recycle les substances de départ n'ayant pas réagi.

2. Procédé selon la revendication 1, caractérisé en ce que le catalyseur est mis en forme avec un liant, puis calciné.

3. Procédé selon la revendication 1 ou 2, caractérisé en ce que le catalyseur est soumis à un traitement par un acide.

4. Procédé selon l'une quelconque des revendications 1 à 3, caractérisé en ce que le catalyseur est dopé avec des métaux de transition.

5. Procédé selon l'une quelconque des revendications 1 à 3, caractérisé en ce que le catalyseur est dopé avec des terres rares.

6. Procédé selon l'une quelconque des revendications 1 à 3, caractérisé en ce que le catalyseur est dopé avec des métaux alcalins, alcalinoterreux et/ou terreux.

7. Procédé selon l'une quelconque des revendications 1 à 3, caractérisé en ce que le catalyseur est utilise, après avoir été traité par des sels d'ammoniac, sous sa forme ammonique.